# EUROPEAN PATENT APPLICATION

(11) **EP 4 059 508 A1**
(43) Date of publication of application: **21.09.2022**
(21) Application number: 21305339.0
(22) Date of filing: 19.03.2021
(51) Int. Cl.: A61K 36/09, A61P 1/00, A61P 17/00, A61P 27/02

(54) **METHODS OF USING COMPOSITIONS COMPRISING AN ICELAND MOSS EXTRACT**

(71) Applicant: Johnson & Johnson Consumer Inc., Skillman, NJ 08558 (US)
(72) Inventor: BRUN, Cecilia, 27100 VAL DE REUIL (FR); ODDOS, Thierry, 27100 VAL DE REUIL (FR); MANGEZ, Claire, 27100 VAL DE REUIL (FR)
(74) Representative: Carpmaels & Ransford LLP

(57) **Abstract**

Provided are methods of reducing pain or itch, the method comprising administering to a patient in need thereof a therapeutically effective dose of a composition comprising a nonpolar extract of Iceland moss.

## Description

### FIELD

The present invention relates to compositions for the reduction of pain and/or itch. More specifically, the present invention relates to compositions comprising an extract of Iceland moss for reducing pain and/or itch, either topically or internally.

### BACKGROUND

A wide variety of natural actives suitable for use in personal care compositions are known. Examples of such actives include olive leaf extract, which has been described as exhibiting antihypertensive and hypoglycemic activities, antiradical properties for alimentary and cosmetics, and anti-inflammatory activity when given via the oral route (see, for example, Leaf extract of Olea europea rich in oleuropeine, products from it, their application as medicines and compositions containing them. Combes, Georges; Escaut, Alexandre. Fr. Demande, FR 2507477 A1 19821217, 1982; Gonzalez M, et al, Hypoglycemic activity of olive leaf, Planta Med December 1992; 58(6):513-515; Use of an extract from the leaves of Olea Europea as an antiradical agent. Amari, Giorgio. Eur. Pat. Appl. (1999), EP 937455 A1 19990825; Fehri B, et al. Olea europaea L.: stimulant, antiulcer and anti-inflammatory effects. Boll Chim Farm (1996) 135(1): 42-49), Sigesbeckia (Holy Herb), which has been used as a remedy for ague, rheumatism, renal colic, and as a cure for ringworm in conjunction with glycerin, Lignum Sappan (Sappan Wood), which has been used to promote blood circulation and remove blood stasis, and to cause subsidence of swelling and relieve pain, and feverfew, recognized as having significant medicinal properties when taken orally and used as a general febrifuge. Other skin care actives include oil extracts a such as Boswellia Serrata oil extract (frankincense), described as exhibiting anti-tumor and anti-arthritic properties, and oat oil extract, described as exhibiting anti-irritant and an antioxidant properties.

Despite increasing demands for actives derived from a natural source, it is generally difficult, if not impossible, to predict ones which have a beneficial property, such as reduction of pain and/or itching. Thus, there is an ongoing need to identify new actives for use in personal care products, such as for use for skin, eye and pain relief.

### SUMMARY OF THE INVENTION

One aspect of the invention pertains to a method of reducing pain or itch, the method comprising administering to a patient in need thereof a therapeutically effective dose of a composition comprising a non-polar extract of Iceland moss. Another aspect of the invention pertains a composition comprising a therapeutically effective dose of non-polar extract of Iceland moss for use in reducing pain or itch to a patient in need thereof. Another aspect of the invention pertains to use of a therapeutically effective dose of a composition comprising a non-polar extract of Iceland moss for reducing pain or itch in a patient in need thereof. Another aspect of the invention pertains to use of a therapeutically effective dose of a non-polar extract of Iceland moss for the preparation of a composition according to any of the preceding claims, to reduce pain or itch in a patient in need thereof.

In one or more embodiments, the non-polar extract was extracted with one or more solvents selected from the group consisting of C1-C8 alkanes, C1-C8 cycloalkanes, C1-C8 alkyl ethers, petroleum ethers, C1-C8 ketones, methylene chloride, ethyl acetate, xylene, toluene, chloroform, vegetable oil, mineral oil, and combinations thereof. In some embodiments, the non-polar extract was extracted with dichloromethane. In one or more embodiments, the pain is TRPA1 receptor-mediated pain and/or itch. In some embodiments, the pain is TrKA receptor-mediated pain and/or itch. In one or more embodiments, the composition is applied to a surface of skin of the patient. In some embodiments, the pain or itch is associated with digestive health, ocular itch, osteoarthritis, allergy, or atopic dermatitis. In one or more embodiments, the composition further comprises a dermatologically acceptable carrier selected from the group consisting of surfactants, chelating agents, emollients, humectants, conditioners, preservatives, opacifiers, fragrances, and combinations of two or more thereof. In some embodiments, the composition is administered in the form of a solution, suspension, lotion, cream, serum, gel, stick, spray, ointment, liquid wash, soap bar, shampoo, hair conditioner, paste, foam, powder, mousse, shaving cream, hydrogel, or film-forming product. In one or more embodiments, the composition is administered enterally. In some embodiments, the composition is administered in the form of a tablet, pill, capsule, caplet, gelcap, lozenge, granule, powder, sterile parenteral solution or suspension, metered aerosol or liquid spray, drop, ampoule, autoinjector device or suppository. In one or more embodiments, the composition is administered to the eye. In some embodiments, the composition is administered in the form of an eye drop solution, eye wash solution, contact lens lubricating and/or rewetting solution, spray, or mist. In one or more embodiments, the non-polar extract is present in the composition at a concentration of about 0.00001 to about 2 wt.% by weight of the total composition. In some embodiments, the non-polar extract is present in the composition at a concentration of about 0.001 to about 0.005 wt.% by weight of the total composition. Such embodiments can be joined in any combination for any of the aspects described herein.

Another aspect of the invention pertains to a method of reducing pain or itch, the method comprising administering to a patient in need thereof a therapeutically effective dose of a composition comprising a non-polar extract of Iceland moss, wherein the non-polar extract was extracted with dichloromethane. Another aspect of the invention pertains to use of a therapeutically effective dose of a composition comprising a non-polar extract of Iceland moss, wherein the non-polar extract was extracted with dichloromethane, for the reduction of pain or itch.

In one or more embodiments, the pain is TRPA1 receptor-mediated pain and/or itch. In some embodiments, the pain is TrKA receptor-mediated pain and/or itch. In one or more embodiments, the composition is applied to a surface of skin of the patient. In some embodiments, the pain or itch is associated with digestive health, ocular itch, osteoarthritis, allergy, or atopic dermatitis. In one or more embodiments, the composition further comprises a dermatologically acceptable carrier selected from the group consisting of surfactants, chelating agents, emollients, humectants, conditioners, preservatives, opacifiers, fragrances, and combinations of two or more thereof. In some embodiments, the composition is administered in the form of a solution, suspension, lotion, cream, serum, gel, stick, spray, ointment, liquid wash, soap bar, shampoo, hair conditioner, paste, foam, powder, mousse, shaving cream, hydrogel, or film-forming product. In one or more embodiments, the composition is administered enterally. In some embodiments, the composition is administered in the form of a tablet, pill, capsule, caplet, gelcap, lozenge, granule, powder, sterile parenteral solution or suspension, metered aerosol or liquid spray, drop, ampoule, autoinjector device or suppository. In one or more embodiments, the composition is administered to the eye. In some embodiments, the composition is administered in the form of an eye drop solution, eye wash solution, contact lens lubricating and/or rewetting solution, spray, or mist. Such embodiments can be joined in any combination for any of the aspects described herein.

These and other features and advantages of the present invention will be readily apparent from the following detailed description of the invention.

### DETAILED DESCRIPTION OF THE INVENTION

As used herein, "dermatologically acceptable" "ophthalmologically acceptable" or "pharmaceutically acceptable" means suitable for use in contact with tissues (e.g., the skin or hair) without undue toxicity, incompatibility, instability, irritation, and/or allergic response, and the like.

As used herein, the term "safe and effective amount" means an amount sufficient to induce the desired effect, but low enough to avoid serious side effects. The safe and effective amount of the compound, extract, or composition will vary with, *e.g.,* the age, health and environmental exposure of the end user, the duration and nature of the treatment, the specific extract, ingredient, or composition employed, the particular pharmaceutically-acceptable carrier utilized, and like factors.

As used herein, "essentially free" or "substantially free" of an ingredient means containing less than 0.1 weight percent, or less than 0.01 weight percent, or none of an ingredient.

To provide a more concise description, some of the quantitative expressions given herein are not qualified with the term "about". It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

To provide a more concise description, some of the quantitative expressions herein are recited as a range from about amount X to about amount Y. It is understood that wherein a range is recited, the range is not limited to the recited upper and lower bounds, but rather includes the full range from about amount X through about amount Y, or any amount or range therein.

Accordingly, one aspect of the invention pertains to a method of reducing pain and/or itch, the method comprising administering to a patient in need thereof a therapeutically effective dose of a composition comprising a non-polar extract of Iceland moss. Another aspect of the invention pertains to use of a non-polar extract of Iceland moss for reduction of pain and/or itch. It has been surprisingly discovered that such compositions comprising extracts of non-polar Iceland moss provide unexpectedly good anti-pain and/or anti-itch properties. The pain or itch can be associated with a wide variety of conditions, including, but not limited to, diseases of the skin, eye, digestive system, musculoskeletal system and allergic reactions.

In one or more embodiments, the pain and/or itch may be TRPA1 receptor-mediated pain and/or itch. TRPA1 (also known as "transient receptor potential ankyrin 1" and "transient receptor potential cation channel, subfamily A, member 1") is an ion channel located on the plasma membrane of many human and animal cells, and is a sensor for pain, cold and itch.

In one or more embodiments, the pain and/or itch may be TrKA receptor-mediated pain and/or itch. TrKA is the receptor for the Nerve Growth Factor (NGF). TrKA is responsible for neuronal differentiation and growth and avoidance of programmed cell death of nerve cells. In the body, primary afferent nerve fibers have their cell body in the dorsal root ganglia (DRG) and transmit sensory information from the periphery, the organs to the spinal cord and finally the brain. Approximately 40% of DRG cells are TrKA positive and innervate the skin, viscera, muscles, and bones. Inhibition of the NGF/TrKA pathway is a validated target for acute and chronic pain/itch.

### Iceland Moss Extract

Iceland moss (scientific name *Cetraria islandica*) is a lichen which grows in Iceland, northern Europe and northern North America. As used herein "Iceland moss extract" refers to an extract of the entire lichen.

Any suitable manner of preparing the extracts for use in accordance with the present invention may be used. Suitable extracts may be obtained using conventional methods including, but not limited to, direct extraction from the biomass by grinding, macerating, pressing, squeezing, mashing, centrifuging, and/or processes such as cold percolation, agitation/distillation, microwave assisted extraction, sonication, supercritical/subcritical CO₂ compressed gas extraction with or without polarity modifier, pressurized solvent extraction, accelerated solvent extraction, surfactant assisted pressurized hot water extraction, oil extraction, membrane extraction, Soxhlet extraction, the gold finger distillation/extraction and/or processes disclosed, for example, in US Pat. Nos. 7,442,391, 7,473,435, and 7,537,791 to Integrated Botanical Technologies, LLC, incorporated herein by reference, and the like, or by other methods such as solvent extraction, and the like. In particular, an extract in accordance with the present invention may be a solvent-based extraction made by grinding or macerating lichen material in a solvent, typically an organic solvent such as an dichloromethane, alcohol, acetone, liquid carbon dioxide with or without polarity modifier, hexane, or chloroform. The resulting extract comprises mainly organic and/or non-polar compounds. The lichen biomass may be separated entirely from the extraction, and not used after extraction.

Suitable solvents to produce non-polar extracts include solvents such as alkanes, including C₁-C₈ alkanes, cycloalkanes, including C₁-C₈ alkanes, alkyl ethers, including C₁-C₈ alkyl ethers, Petroleum ethers, ketones, including C₁-C₈ ketones, methylene chloride (also known as dichloromethane), ethyl acetate, xylene, toluene, chloroform, vegetable oil, mineral oil and the like. In some embodiments, the non-polar extract is obtained by using the solvents described above or supercritical fluid extraction with or without a polar modifier such as C₁-C₈ alcohols, water, C₁-C₈ polyols/glycols or C₁-C₈ organic acids. In some embodiments, the composition may comprise extracts from cell cultures of Iceland moss. In some embodiments, the non-polar Iceland moss extract was extracted with dichloromethane. In some embodiments, the Iceland moss extract is substantially free of polar extracts, including those extracted using water, C₁-C₈ alcohols, C₁-C₈ polyols, C₁-C₈ glycols and combinations thereof.

Any suitable amount of non-polar Iceland moss extract may be used in the compositions of the present invention. In one or more embodiments, the compositions comprise a safe and effective amounts of non-polar Iceland moss extract. In one or more embodiments, the compositions comprise from about 0.00001, 0.00005, 0.0001, 0.0005, 0.001, 0.002, 0.003, or 0.004 to about 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9 or 2 wt.% non-polar Iceland moss extract. In some embodiments, the non-polar Iceland moss extract is present in the composition at a concentration of about 0.00001 to about 2 wt.% by weight of the total composition. In some embodiments, the non-polar Iceland moss extract is present in the composition at a concentration of about 0.0001to about 1.5 wt.% by weight of the total composition. In some embodiments, the non-polar Iceland moss extract is present in the composition at a concentration of about 0.0005 to about 1 wt.% by weight of the total composition. In some embodiments, the non-polar Iceland moss extract is present in the composition at a concentration of about 0.0005 to about 0.01 wt.% by weight of the total composition. In some embodiments, the non-polar Iceland moss extract is present in the composition at a concentration of about 0.001 to about 0.005 wt.% by weight of the total composition.

### Dermatological Uses

The non-polar Iceland moss extract may be used for pain and/or itch on the skin. In such embodiments the composition is applied to a surface of skin of the patient. Relevant skin conditions which may be treated include, but are not limited to, inflammatory dermatoses, contact dermatitis, allergic dermatitis, atopic dermatitis, polymorphous light eruptions, irritation, including pain/itch induced by extrinsic factors, acne inflammation, psoriasis, seborrheic dermatitis, eczema, poison ivy, poison oak, poison sumac, insect bites, folliculitis, alopecia, and secondary conditions and the like.

Any suitable carrier may be used in the compositions as the context dictates. In some embodiments, the carrier is a dermatologically acceptable carrier. As will be recognized by those of skill in the art, dermatologically acceptable carriers comprise carriers that are suitable for use in contact with the body, in particular the skin, without undue toxicity, incompatibility, instability, irritation, allergic response, and the like. A safe and effective amount of dermatologically carrier is from about 50, 55, 60, 65, 70, 75, 80, 85, 90, 95, or 98% to about 85, 90, 95, 98, 99, 99.1, 99.5 or 99.9% by weight of the composition.

The dermatologically carrier can be in a wide variety of forms. For example, dermatologically carriers in the form of emulsions, including, but not limited to, oil-in-water, water-in-oil, water-in-oil-in-water, and oil-in-water-in-silicone emulsions, are useful herein. These emulsions can cover a broad range of viscosities, e.g., from about 100 cps to about 200,000 cps using a Brookfield RVT viscometer.

Examples of suitable dermatologically acceptable carriers include dermatologically acceptable solvents and materials for dermatological solutions, suspensions, lotions, creams, serums, essences, gels, toners, sticks, sprays, ointments, liquid washes and soap bars, shampoos, hair conditioners, pastes, foams, mousses, powders, shaving creams, wipes, patches, strips, powered patches, micro-needle patches, bandages, hydrogels, film-forming products, facial and skin masks, make-up, liquid drops, and the like. These product types may contain several types of dermatologically acceptable carriers including, but not limited to solutions, suspensions, emulsions such as micro-emulsions and nano-emulsions, gels, solids, liposomes, other encapsulation technologies and the like.

The following are non-limiting examples of dermatologically acceptable carriers. Other dermatologically acceptable carriers can be formulated by those of ordinary skill in the art. In one embodiment, the dermatologically carrier contains water. In a further embodiment, the dermatologically acceptable carrier may also contain one or more aqueous or organic solvents. Examples of organic solvents include, but are not limited to: dimethyl isosorbide; isopropyl myristate; surfactants of cationic, anionic and nonionic nature; vegetable oils; mineral oils; waxes; gums; synthetic and natural gelling agents; alkanols; glycols; and polyols. Examples of glycols include, but are not limited to, glycerin, propylene glycol, butylene glycol, pentalene glycol, hexylene glycol, polyethylene glycol, polypropylene glycol, diethylene glycol, triethylene glycol, capryl glycol, glycerol, butanediol and hexanetriol, and copolymers or mixtures thereof. Examples of alkanols include, but are not limited to, those having from about 2 carbon atoms to about 12 carbon atoms (*e.g.*, from about 2 carbon atoms to about 4 carbon atoms), such as isopropanol and ethanol. Examples of polyols include, but are not limited to, those having from about 2 carbon atoms to about 15 carbon atoms (*e.g.*, from about 2 carbon atoms to about 10 carbon atoms) such as propylene glycol. The organic solvents may be present in the dermatologically acceptable carrier in an amount, based upon the total weight of the dermatologically acceptable carrier, of from about 1 percent to about 99.99 percent (*e.g.,* from about 20 percent to about 50 percent). Water may be present in the dermatologically acceptable carrier (prior to use) in an amount, based upon the total weight of the dermatologically acceptable carrier, of from about 5 percent to about 95 percent (e.g., from about 50 percent to about 90 percent). Solutions may contain any suitable amounts of solvent, including from about 40 to about 99.99%. Some solutions contain from about 50 to about 99.9%, from about 60 to about 99%, from about 70 to about 99%, from about 80 to about 99%, or from about 90 to 99% of solvent.

A lotion can be made from such a solution. Lotions typically contain at least one emollient in addition to a solvent. Lotions may comprise from about 1% to about 20% (*e.g.,* from about 5% to about 10%) of an emollient(s) and from about 50% to about 90% (e.g., from about 60% to about 80%) of water.

Another type of product that may be formulated from a solution is a cream. A cream typically contains from about 5% to about 50% (*e.g.,* from about 10% to about 20%) of an emollient(s) and from about 45% to about 85% (*e.g.,* from about 50% to about 75%) of water.

Yet another type of product that may be formulated from a solution is an ointment. An ointment may contain a simple base of animal, vegetable, or synthetic oils or semi-solid 10 hydrocarbons. An ointment may contain from about 2% to about 10% of an emollient(s) plus from about 0.1% to about 2% of a thickening agent(s).

The compositions useful in the present invention can also be formulated as emulsions. If the dermatologically acceptable carrier is an emulsion, from about 1% to about 10% (*e.g.,* from about 2% to about 5%) of the dermatologically acceptable carrier contains an emulsifier(s). Emulsifiers may be nonionic, anionic or cationic.

Lotions and creams can be formulated as emulsions. Typically such lotions contain from 0.5% to about 5% of an emulsifier(s), while such creams would typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type, and water-in-oil type are well-known in the art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The compositions of this invention can also be formulated as a gel (*e.g.,* an aqueous, alcohol, alcohol/water, or oil gel using a suitable gelling agent(s)). Suitable gelling agents for aqueous and/or alcoholic gels include, but are not limited to, natural gums, acrylic acid and acrylate polymers, and copolymers, and cellulose derivatives (*e.g.*, hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contains between about 0.1% and 5%, by weight, of such gelling agents.

The compositions of the present invention can also be formulated into a solid formulation (*e.g.*, a wax-based stick, soap bar composition, powder, or wipe). The composition of the present invention can also be combined with a solid, semi-solid, or dissolvable substrate (*e.g.*, a wipe, mask, pad, glove, or strip).

### Other Dermatologically Acceptable Additives

The compositions of the present invention may further comprise any of a variety of additional dermatologically acceptable active agents. Examples of suitable additional dermatologically acceptable active agents include: skin lightening agents, darkening agents, additional anti-aging agents, tropoelastin promoters, collagen promoters, anti-acne agents, shine control agents, anti-microbial agents such as anti-yeast agents, antifungal, and anti-bacterial agents, anti-inflammatory agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, hair growth enhancing agents, hair growth delaying agents, firming agents, hydration boosters, efficacy boosters, anti-callous agents, agents for skin conditioning, anti-cellulite agents, odor-control agents such as odor masking or pH changing agents, and the like.

Examples of various suitable additional dermatologically acceptable acceptable actives include hydroxy acids; benzoyl peroxide; D-panthenol; UV filters such as but not limited to avobenzone (Parsol 1789), bisdisulizole disodium (Neo Heliopan AP), diethylamino hydroxybenzoyl hexyl benzoate (Uvinul A Plus), ecamsule (Mexoryl SX), methyl anthranilate, 4-aminobenzoic acid (PABA), cinoxate, ethylhexyl triazone (Uvinul T 150), homosalate, 4-methylbenzylidene camphor (Parsol 5000), octyl methoxycinnamate (Octinoxate), octyl salicylate (Octisalate), padimate O (Escalol 507), phenylbenzimidazole sulfonic acid (Ensulizole), polysilicone-15 (Parsol SLX), trolamine salicylate, Bemotrizinol (Tinosorb S), benzophenones 1-12, dioxybenzone, drometrizole trisiloxane (Mexoryl XL), iscotrizinol (Uvasorb HEB), octocrylene, oxybenzone (Eusolex 4360), sulisobenzone, bisoctrizole (Tinosorb M), titanium dioxide, zinc oxide; carotenoids; free radical scavengers; spin traps; retinoids and retinoid precursors such as retinol, retinoic acid and retinyl palmitate; ceramides; polyunsaturated fatty acids; essential fatty acids; enzymes; enzyme inhibitors; minerals; hormones such as estrogens; steroids such as hydrocortisone; 2-dimethylaminoethanol; copper salts such as copper chloride; peptides containing copper such as Cu:Gly-His-Lys, coenzyme Q10; amino acids such a proline; vitamins; lactobionic acid; acetyl-coenzyme A; niacin; riboflavin; thiamin; ribose; electron transporters such as NADH and FADH2; and other botanical extracts such as oat, aloe vera, Feverfew, Soy, Shiitake mushroom extracts, and derivatives and mixtures thereof.

If present, any additional dermatologically acceptable active agent may be present in a composition in any suitable amount, for example, in an amount of from about 0.0001% to about 20% by weight of the composition, e.g., about 0.001% to about 10% such as about 0.01% to about 5%. In some embodiments, in an amount of 0.1% to 5% and in other embodiments from 1% to 2%.

Compositions of the present invention may include a dermatologically acceptable effective amount of one or more additional anti-inflammatory compounds. Examples of suitable anti-inflammatory agents include substituted resorcinols, (E)-3-(4-methylphenylsulfonyl)-2-propenenitrile (such as "Bay 11-7082," commercially available from Sigma-Aldrich of St. Louis, Missouri), tetrahydrocurcuminoids (such as Tetrahydrocurcuminoid CG, available from Sabinsa Corporation of Piscataway, NJ), extracts and materials derived from the following: *Phellodendron amurense* Cortex Extract (PCE), Non-Denatured Soy (*Glycine max*), Feverfew (*Tanacetum parthenium*), Ginger (*Zingiber officinale*), Ginkgo (*Ginkgo biloba*), Madecassoside (*Centella asiatica* extract ingredient), Cotinus (*Cotinus coggygria*), Butterbur Extract (*Petasites hybridus*), Goji Berry (*Lycium barbarum*), Milk Thistle Extract (*Silybum marianum*), Honeysuckle (*Lonicera japonica*), Basalm of Peru (*Myroxylon pereirae*), Sage (*Salvia officinalis*), Cranberry Extract (*Vaccinium oxycoccos*), Amaranth Oil (*Amaranthus cruentus*), Pomegranate (*Punica granatum*), Yerbe Mate (*Ilex paraguariensis* Leaf Extract), White Lily Flower Extract (*Lilium candidum*), Olive Leaf Extract (*Olea europaea*), Phloretin (apple extract), Oat Flour (*Aveena sativa*), Lifenol (Hops: *Humulus lupulus*) Extract, Bugrane P (*Ononis spinosa*), Licochalcone (Licorice: *Glycyrrhiza inflate* extract ingredient), Symrelief (Bisabolol and Ginger extract), combinations of two or more thereof, and the like.

One example of a dermatologically acceptable anti-inflammatory agent is a resorcinol. Particularly suitable substituted resorcinols include 4-hexyl resorcinol and 4-octylresorcinol, particularly 4-hexyl resorcinol. 4-Hexyl resorcinol is commercially available as "SYNOVEA HR" from Sytheon of Lincoln Park, NJ. 4-Octylresorcinol is commercially available from City Chemical LLC of West Haven, Connecticut.

By "extracts of feverfew," it is meant extracts of the plant "*Tanacetum parthenium,"* such as may be produced according to the details set for the in US Patent No. 7,537,791, entitled "PARTHENOLIDE FREE BIOACTIVE INGREDIENTS FROM FEVERFEW (TANACETUM PARTHENIUM) AND PROCESSES FOR THEIR PRODUCTION." One particularly suitable feverfew extract is commercially available as about 20% active feverfew, from Integrated Botanical Technologies of Ossining, NY.

A variety of other dermatologically acceptable materials may also be present in the compositions of the present invention. In one or more embodiments, the composition comprises one or more topical ingredients selected from the group consisting of: surfactants, chelating agents, emollients, humectants, conditioners, preservatives, opacifiers, fragrances and the like.

What is meant by an emollient is a compound that helps to maintain the soft, smooth, and pliable appearance of the skin (*e.g.,* by remaining on the skin surface or in the stratum corneum to act as a lubricant). Examples of suitable emollients include those found in Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY), and include, but are not limited to, petrolatum, hexyldecyl stearate and plant, nut, and vegetable oils such as macadamia nut oil, rice bran oil, grape seed oil, palm oil, prim rose oil, hydrogenates peanut oil, and avocado oil.

What is meant by a humectant is a compound intended to increase the water content of the top layers of skin (*e.g.*, hygroscopic compounds). Examples of suitable humectants include those found in Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY) and include, but are not limited to, glycerin, sorbitol or trehalose (*e.g.*, α,α- trehalose, β,β-trehalose, α,β-trehalose) or a salt or ester thereof (*e.g.*, trehalose 6-phosphate).

What is meant by a surfactant is a surface-active agent intended to cleanse or emulsify. Examples of suitable surfactants include those found in Chapter 37, pages 431-450 (Classification of surfactants, by L. Oldenhove de Guertechin) in Handbook of Cosmetic Science and Technology (edited by A. Barel, M. Paye and H. Maibach, Published in 2001 by Marcel Dekker, Inc., New York, NY) and include, but are not limited to anionic surfactants such as sulfates and sodium coco glycinate, cationic surfactants, amphoteric surfactants such as betaines, nonionic surfactants such as alkyl polyglucosides.

Examples of suitable dermatologically acceptable chelating agents include those which are capable of protecting and preserving the compositions of this invention. In one or more embodiments, the chelating agent is ethylenediaminetetraacetic acid ("EDTA"), and more specifically is tetrasodium EDTA, available commercially from Dow Chemical Company of Midland, Michigan under the trade name, "Versene 100XL."

Suitable dermatologically acceptable preservatives include, for example, parabens, quaternary ammonium species, phenoxyethanol, benzoates, DMDM hydantoin, organic acids and are present in the composition in an amount, based upon the total weight of the composition, from about 0 to about 1 percent or from about 0.05 percent to about 0.5 percent.

Any of a variety of conditioners which impart additional attributes, such as gloss to the hair, are suitable for use in this invention. Examples include, but are not limited to, volatile silicone conditioning agent having an atmospheric pressure boiling point less than about 220°C. Examples of suitable volatile silicones nonexclusively include polydimethylsiloxane, polydimethylcyclosiloxane, hexamethyldisiloxane, cyclomethicone fluids such as polydimethylcyclosiloxane available commercially from Dow Corning Corporation of Midland, Michigan under the tradename, "DC-345" and mixtures thereof, and specifically include cyclomethicone fluids. Other suitable conditioners include cationic polymers, including polyquarterniums, cationic guar, and the like.

Any of a variety of commercially available pearlescent or opacifying agents are suitable for use in the composition. Examples of suitable pearlescent or opacifying agents include, but are not limited to, mono or diesters of (a) fatty acids having from about 16 to about 22 carbon atoms and (b) either ethylene or propylene glycol; mono or diesters of (a) fatty acids having from about 16 to about 22 carbon atoms (b) a polyalkylene glycol of the formula: HO-(JO)a-H, wherein J is an alkylene group having from about 2 to about 3 carbon atoms; and a is 2 or 3; fatty alcohols containing from about 16 to about 22 carbon atoms; fatty esters of the formula: KCOOCH₂L, wherein K and L independently contain from about 15 to about 21 carbon atoms; inorganic solids insoluble in the shampoo composition, and mixtures thereof.

Any fragrance compositions suitable for use on skin may be used in the composition according to the present invention.

### Dermatological Product Forms

The composition may be applied topically. Such topical application may be to any skin in need of treatment on the body, for example skin of the face, lips, neck, chest, back, buttocks, arms, axilla, and/or legs. The composition may also be administered to a mucous membrane (*i.e.,* in the oral cavity).

In some embodiments, the present invention is in the form of a substrate comprising a composition of the present invention. Any suitable substrate may be used. Examples of suitable substrates and substrate materials are disclosed, for example, in US7452547 and US2009/0241242 which are incorporated herein by reference in their entirety. In some embodiments, the composition is in the form of a tablet, pill, or capsule. In one or more embodiments, the composition is in the form of a solution, suspension, emulsion, lotion, cream, serum, gel, stick, spray, ointment, liquid wash, soap bar, shampoo, hair conditioner, paste, foam, powder, mousse, shaving cream, hydrogel, or film-forming product.

Any suitable method of applying the composition to the skin in need may be used. For example, the composition may be applied directly from a package to the skin in need, by hand to the skin in need, or may be transferred from a substrate such as a wipe or mask, or a combination of two or more thereof. In other embodiments, the composition may be applied via a dropper, tube, roller, spray, and patch or added to a bath or otherwise to water to be applied to the skin, and the like. The composition may be applied in a variety of manners or forms, including, without limitation, as a leave-on cream, mask, and /or serum.

### Ophthalmological Use

The non-polar Iceland moss extract may be used for pain and/or itch in the eye. In such embodiments the composition is administered to the patient. Relevant eye conditions which may be treated include, but are not limited to, ocular itch, conjunctivitis, allergy and pain/itch induced by extrinsic factors.

For ophthalmological use, the extract may be formulated with a ophthalmologically acceptable carrier. Useful ophthalmologically acceptable oil-in-water and water-oil-carriers can be found in US Patent Publication 20030165545A1 and U.S. Pat. Nos. 9,480,645, 8,828,412 and 8,496,976, each of which patent documents are herein incorporated by reference in its entirety.

The ophthalmologically acceptable carrier (or, compositions of the present invention) may optionally comprise one or more additional excipients and/or one or more additional active ingredients. Examples of such optional components are described below.

Excipients commonly used in ophthalmic compositions include, but are not limited to, demulcents, tonicity agents, preservatives, chelating agents, buffering agents (other than and in addition to the organic acids of the present invention), and surfactants. Other excipients comprise solubilizing agents, stabilizing agents, comfort-enhancing agents, polymers, emollients, pH-adjusting agents (other than and in addition to the organic acids of the present invention), and/or lubricants. Any of a variety of ophthalmologically acceptable excipients may be used in the compositions of the present invention including water, mixtures of water and water-miscible solvents, such as vegetable oils or mineral oils comprising from 0.5% to 5% non-toxic water-soluble polymers, natural products, such as agar and acacia, starch derivatives, such as starch acetate and hydroxypropyl starch, and also other synthetic products such as polyvinyl alcohol, polyvinylpyrrolidone, polyvinyl methyl ether, polyethylene oxide, and preferably cross-linked polyacrylic acid and mixtures thereof.

Demulcents or soothing agents used with embodiments of the present invention include, but are not limited to, cellulose derivatives (such hydroxyethyl cellulose, methyl cellulose, hypromellose or mixtures thereof), hyaluronic acid, tamarind seed extract, glycerin, polyvinyl pyrrolidone, polyethylene oxide, polyethylene glycol, propylene glycol and polyacrylic acid and mixtures thereof. In some embodiments, one or more of hyaluronic acid, propylene glycol, tamarind seed extract, glycerin and/or polyethylene glycol 400 are the demulcents or soothing agents. In one or more embodiments, the demulcent or soothing agent is selected from hyaluronic acid, tamarind seed extract or mixtures thereof.

In some embodiments, compositions of the present invention are ophthalmologically suitable for application to a subject's eyes. The term "aqueous" typically denotes an aqueous formulation wherein the excipient is > about 50%, more preferably > about 75% and in particular > about 90% by weight water. In one or more embodiments, the compositions of the present invention are essentially free of compounds which irritate the eye. In some embodiments, the compositions of the present invention are essentially free of free fatty acids and C1 to C4 alcohols. In one or more embodiments, the compositions of the present invention are comprise less than 40% (or about 40%), optionally, less than 35% (or about 35%), optionally, less than 30% (or about 30%), optionally less than 25% (or about 25%), optionally, less than 20% (or about 20%), optionally, less than 15% (or about 15%), optionally less than 10% (or about 10%), or optionally, less than 5% (or about 5%), by weight of the total composition, of a non-alcohol, organic excipient or solvent. These drops may be delivered from a single dose ampoule which may preferably be sterile and thus render bacteriostatic components of the formulation unnecessary. Alternatively, the drops may be delivered from a multi-dose bottle which may preferably comprise a device which extracts any preservative from the composition as it is delivered, such devices being known in the art.

In one or more embodiments, the compositions of the present invention are isotonic, or slightly hypotonic in order to combat any hypertonicity of tears caused by evaporation and/or disease. This may require a tonicity agent to bring the osmolality of the formulation to a level at or near 210-320 milliosmoles per kilogram (mOsm/kg). In some embodiments, the compositions of the present invention generally have an osmolality in the range of 220-320 mOsm/kg, or, optionally, have an osmolality in the range of 235-300 mOsm/kg. The ophthalmic compositions will generally be formulated as sterile aqueous solutions.

The osmolality of the ophthalmic compositions may be adjusted with tonicity agents to a value which is compatible with the intended use of the compositions. For example, the osmolality of the ophthalmic composition may be adjusted to approximate the osmotic pressure of normal tear fluid, which is equivalent to about 0.9 w/v % of sodium chloride in water. Examples of suitable tonicity adjusting agents include, without limitation, sodium, potassium, calcium and magnesium chloride; dextrose; glycerin; propylene glycol; mannitol; sorbitol and the like and mixtures thereof. In one embodiment, a combination of sodium chloride and potassium chloride are used to adjust the tonicity of the composition.

Ophthalmic compositions can also be used to administer pharmaceutically active compounds. Such compounds include, but are not limited to, glaucoma therapeutics, pain relievers, anti-inflammatory and anti-allergy medications, and anti-microbials. More specific examples of pharmaceutically active compounds include betaxolol, timolol, pilocarpine, carbonic anhydrase inhibitors and prostglandins; dopaminergic antagonists; post-surgical antihypertensive agents, such as para-amino clonidine (apraclonidine); antiinfectives such as ciprofloxacin, moxifloxacin, and tobramycin; non-steroidal and steroidal anti-inflammatories, such as naproxen, diclofenac, nepafenac, suprofen, ketorolac, tetrahydrocortisol and dexamethasone; dry eye therapeutics such as PDE4 inhibitors; and anti-allergy medications such as H1/H4 inhibitors, H4 inhibitors, olopatadine or mixtures thereof.

In some embodiments, the ophthalmic compositions may have a pH which is compatible with the intended use, and is often in the range of 4 (or about 4) to 10 (or about 10), optionally between 6 (or about 6) to 8 (to about 8), optionally between 6.5 (or about 6.5) to 7.5 (or about 7.5), or optionally between 6.8 (or about 6.8) to 7.2 (or about 7.2).

In one or more embodiments, a variety of conventional buffers may be employed, such as phosphate, borate, citrate, acetate, histidine, tris, bis-tris and the like and mixtures thereof. Borate buffers include boric acid and its salts, such as sodium or potassium borate. Potassium tetraborate or potassium metaborate, which produce boric acid or a salt of boric acid in solution, may also be employed. Hydrated salts such as sodium borate decahydrate can also be used. Phosphate buffers include phosphoric acid and its salts; for example, M2HPO4 and MH2PO4, wherein M is an alkali metal such as sodium and potassium. Hydrated salts can also be used. In one embodiment of the present invention, Na2HPO4.7H2O and NaH2PO2.H2O are used as buffers. The term phosphate also includes compounds that produce phosphoric acid or a salt of phosphoric acid in solution. Additionally, organic counter-ions for the above buffers may also be employed. The concentration of buffer generally varies from about 0.01 to 2.5 w/v % and more preferably varies from about 0.05 to about 0.5 w/v %.

In one or more embodiments, the viscosity of the compositions of the present invention range from about 1 to about 500 cps, optionally from about 10 to about 200 cps, or optionally from about 10 to about 100 cps, when measured using a TA Instrument AR 2000 rheometer. The TA Instrument AR 2000 rheometer should be used with the AR2000 flow test method of the TA Rheological Advantage software with a 40 mm steel plate geometry; the viscosity ranges should be obtained by measuring steady state flow controlling shear rate from 0 sec⁻¹ to 200 sec⁻¹.

In one or more embodiments, the compositions may be administered in the form of, eye drop solution, eye wash solution, contact lens lubricating and/or rewetting solution, spray, mist or any other manner of administering a composition to the eye.

The compositions of the present invention may be in the form of packing solutions for contact lenses. In one or more embodiments, as packing solutions, the compositions of the present invention may be sealed in blister packaging and, also, suitable for undergoing sterilization.

Examples of blister packages and sterilization techniques are disclosed in the following references which are hereby incorporated by reference in their entirety, U.S. Pat. Nos. D435,966; 4,691,820; 5,467,868; 5,704,468; 5,823,327; 6,050,398, 5,696,686; 6,018,931; 5,577,367; and 5,488,815. This portion of the manufacturing process presents another method of treating the ophthalmic devices with anti-allergic agent, namely adding anti-allergic agents to a solution prior to sealing the package, and subsequently sterilizing the package. This is the preferred method of treating ophthalmic devices with anti-allergic agents.

Sterilization can take place at different temperatures and periods of time. The preferred sterilization conditions range from about 100° C. for about 8 hours to about 150° C. for about 0.5 minute. More preferred sterilization conditions range from about 115° C. for about 2.5 hours to about 130° C. for about 5.0 minutes. The most preferred sterilization conditions are about 124° C. for about 18 minutes.

When used as packing solutions, the compositions of the present invention may be water-based solutions. Typical packing solutions include, without limitation, saline solutions, other buffered solutions, and deionized water. In one or more embodiments, the packing solution is an aqueous solution of deioinized water or saline solution containing salts including, without limitation, sodium chloride, sodium borate, sodium phosphate, sodium hydrogenphosphate, sodium dihydrogenphosphate, or the corresponding potassium salts of the same. These ingredients are generally combined to form buffered solutions that include an acid and its conjugate base, so that addition of acids and bases cause only a relatively small change in pH. In one or more embodiments, the pH of the packing solution is as described above. The buffered solutions may additionally include 2-(N-morpholino)ethanesulfonic acid (MES), sodium hydroxide, 2,2-bis(hydroxymethyl)-2,2',"-nitrilotriethanol, n-tris(hydroxymethyl)methyl-2-aminoethanesulfonic acid, citric acid, sodium citrate, sodium carbonate, sodium bicarbonate, acetic acid, sodium acetate, ethylenediamine tetraacetic acid and the like and combinations thereof. Preferably, the solution is a borate buffered or phosphate buffered saline solution or deionized water. The particularly preferred solution contains about 500 ppm to about 18,500 ppm sodium borate, most particularly preferred about 1000 ppm of sodium borate.

If any ingredients incorporated into the packing solutions are subject to oxidative degradation, agents that stabilize packing solutions containing such ingredients may be added. Such "oxidative stabilization agents" include but are not limited to chelants such as EDTA, Dequest, Desferal, silica, chitin derivatives such as chitosan, cellulose and its derivatives, and N,N,N',N',N", N"-hexa(2-pyridyl)-1,3,5-tris(aminomethyl)benzene, and certain macrocyclic ligands such as crown ethers, ligand containing knots and catenands. See, David A. Leigh et al Angew. Chem Int. Ed., 2001, 40, No. 8, pgs. 1538-1542 and Jean-Claude Chambron et al. Pure & Appl. Chem., 1990, Vol. 62, No. 6, pgs. 1027-1034. Oxidative stabilization agents may include other compounds that inhibit oxidations such as those selected from the group consisting of 2,2',2',6,6',6"-Hexa-(1,1-dimethylethyl)4,4',4"-[(2,4,6-trimethyl-1,3,5-benzenetriyl)-trismethylene]-triphenol (Irganox 1330), 1,3,5tris[3,5-di(1,1-dimethylethyl)4-hydroxybenzyl]-1H,3H,5H-1,3,5-triazine-2,4,6-trione, pentaerythrityl tetrakis[3-[3,5-di(1,1-dimethylethyl)-4-hydroxyphenyl]-propionate], octadecyl-3-[3,5-di(1,1-dimethylethyl)-4-hydroxyphenyl]-propionate, tris[2,4-di(1,1-dimethylethyl)-phenyl]-phosphite, 2,2'-di(octadecyloxy)-5,5'-spirobi(1,3,2-dioxaphosphorinane), dioctadecyl disulphide, didodecyl-3,3'-thiodipropionate, dioctadecyl-3,3'-thiodipropionate, butylhydroxytoluene, ethylene bis[3,3-di[3-(1,1-dimethylethyl)-4-hydroxyphenyl]butyrate] and mixtures thereof. The preferred oxidative stabilization agents are diethylenetriaminepentaacetic acid ("DTPA"), or salts of DTPA such as CaNa₃DTPA, ZnNa₃DTPA, and Ca₂DTPA. See, U.S. App. Pat. No. 60/783,557 filed on, Mar. 17, 2006, entitled "Methods for Stabilizing Oxidatively Unstable Pharmaceutical Compositions" and its corresponding non-provisional filing which are hereby incorporated by reference in their entirety. In one or more embodiments, the concentration of oxidative stabilization agents in the solution be from about 2.5 µmoles/liter to about, 5000 µmoles/liter, optionally, from about 20 µmoles/liter to about 1000 µmoles/liter, optionally from about 100 µmoles/liter to about 1000 µmoles/liter, or optionally from about 100 µmoles/liter to about 500 µmoles/liter.

In one or more embodiments, the ophthalmic compositions are formulated for administration at any frequency of administration, including once a week, once every five days, once every three days, once every two days, twice a day, three times a day, four times a day, five times a day, six times a day, eight times a day, every hour, or greater frequency. Such dosing frequency is also maintained for a varying duration of time depending on the therapeutic needs of the user. The duration of a particular therapeutic regimen may vary from one-time dosing to a regimen that extends for months or years. One of ordinary skill in the art would be familiar with determining a therapeutic regimen for a specific indication.

### Pharmaceutical Use

The non-polar Iceland moss extract may be used for enteral pain and/or itch on the skin. In such embodiments the composition is administered enterally. Relevant enteral conditions which may be treated include, but are not limited to, conditions related to digestive health (e.g., irritable bowel syndrome, etc.), musculoskeletal system (e.g., osteoarthritis, other muscular pain, etc.).

To prepare a composition for non-topical route of administration of the present invention (referred to herein as "pharmaceutical use"), a non-polar Iceland moss extract may be intimately admixed with a pharmaceutically acceptable carrier according to conventional pharmaceutical compounding techniques, which pharmaceutically acceptable carrier may take a wide variety of forms depending of the form of preparation desired for administration (*e.g.*, oral or parenteral). Suitable pharmaceutically acceptable carriers are well known in the art. Descriptions of some of these pharmaceutically acceptable carriers may be found in The Handbook of Pharmaceutical Excipients, published by the American Pharmaceutical Association and the Pharmaceutical Society of Great Britain.

Methods of formulating pharmaceutical compositions have been described in numerous publications such as Pharmaceutical Dosage Forms: Tablets, Second Edition, Revised and Expanded, Volumes 1-3, edited by Lieberman et al; Pharmaceutical Dosage Forms: Parenteral Medications, Volumes 1-2, edited by Avis et al; and Pharmaceutical Dosage Forms: Disperse Systems, Volumes 1-2, edited by Lieberman et al; published by Marcel Dekker, Inc.

Preferably a pharmaceutical composition is in a unit dosage form such as a tablet, pill, capsule, caplet, gelcap, lozenge, granule, powder, sterile parenteral solution or suspension, metered aerosol or liquid spray, drop, ampoule, autoinjector device or suppository for administration by oral, intranasal, sublingual, transdermal, parenteral, rectal, vaginal, inhalation or insufflation means. Alternatively, the composition may be presented in a form suitable for once-weekly or once-monthly administration or may be adapted to provide a preparation for intramuscular injection.

In preparing a pharmaceutical composition having a solid dosage form for oral administration, such as a tablet, pill, capsule, caplet, gelcap, lozenge, granule or powder (each including immediate release, timed release and sustained release formulations), pharmaceutically suitable carriers and additives include but are not limited to diluents, granulating agents, lubricants, binders, glidants, disintegrating agents and the like. If desired, tablets may be sugar coated, gelatin coated, film coated or enteric coated by standard techniques.

For preparing a solid dosage form, the principal active ingredient is mixed with a pharmaceutically acceptable carrier (e.g. conventional tableting ingredients such as diluents, binders, adhesives, disintegrants, lubricants, antiadherents and glidants). Sweeteners and flavorants may be added to chewable solid dosage forms to improve the palatability of the oral dosage form. Additionally, colorants and coatings may be added or applied to the solid dosage form for ease of identification of the drug or for aesthetic purposes. These pharmaceutically acceptable carriers are formulated with the pharmaceutical active to provide an accurate, appropriate dose of the pharmaceutical active with a therapeutic release profile.

In preparing a pharmaceutical composition having a liquid dosage form for oral, topical and parenteral administration, any of the usual pharmaceutical media or excipients may be employed. Thus, for liquid unit dosage forms, such as suspensions (i.e. colloids, emulsions and dispersions) and solutions, pharmaceutically suitable carriers and additives include but are not limited to pharmaceutically acceptable wetting agents, dispersants, flocculation agents, thickeners, pH control agents (i.e. buffers), osmotic agents, coloring agents, flavors, fragrances, preservatives (i.e. to control microbial growth, etc.) and a liquid vehicle may be employed. Not all of the components listed above will be required for each liquid dosage form. The liquid forms in which the novel compositions of the present invention may be incorporated for administration orally or by injection include, but are not limited to aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles.

While the foregoing description represent exemplary embodiments of the present invention, it will be understood that various additions, modifications and substitutions may be made therein without departing from the spirit and scope of the present invention. In particular, it will be clear to those skilled in the art that the present invention may be embodied in other specific forms, structures, arrangements, proportions, and with other elements, materials, and components, without departing from the spirit or essential characteristics thereof. One skilled in the art will appreciate that the invention may be used with many modifications of structure, arrangement, proportions, materials, and components and otherwise, used in the practice of the invention, which are particularly adapted to specific environments and operative requirements without departing from the principles of the present invention. The presently disclosed embodiments are therefore to be considered in all respects as illustrative and not restrictive, the scope of the invention being indicated by the appended claims, and not limited to the foregoing description. It will be appreciated that in the claims, the term "comprises/comprising" does not exclude the presence of other elements or steps. In addition, singular references do not exclude a plurality. The terms "a", "an", "first", "second", etc., do not preclude a plurality.

All percentages, parts and ratios are based upon the total weight of the composition of the present invention, unless otherwise specified. All such weights as they pertain to the listed ingredients are based on the level of the particular ingredient described and, therefore, do not include carriers or by-products that may be included in commercially available materials, unless otherwise specified.

### EXAMPLES

### EXAMPLE 1 - Polar Versus Non-Polar Extract

Two extracts of Iceland moss were compared for antagonistic activity of the TRPA1 receptor. Both extracts are supplied in DMSO at 10 mg/mL, and are included in the Phytotitre Natural Product Extract Library and available from Caithness Biotechnologies Ltd. One extract was a polar extract (aqueous solution) (comparative) while the other was non-polar (dichloromethane as solvent) (inventive).

### Pre-Screening on TRPA1 Receptor:

The extracts were screened using the Human TRPA1 Ion Channel Cell Line from PerkinElmer. An expression vector containing the coding sequence of the human Transient Receptor Potential cation channel subfamily A member 1 (TRPA1) ion channel under the control of the CMV promoter was transfected in HEK-293 cells stably expressing mitochondrially targeted Photina^{®}.

### Experimental protocol:

At Day 0, 80.000 cells /wells were seeded in 100 µL of culture medium (MEM: Minimum Essential Medium Eagle medium +10% Fetal bovine serum + antibiotic G418) and then incubated overnight at 37°C.

At Day 1, the medium was discarded and 100 µL of coelenterazine was added in each well and the cells were incubated for 4 hours at 25°C. Then, 25 µl of each Iceland moss extract at either 0.001 % or 0.005% were added. Ruthenium red (Sigma Aldrich) at 50µM was used as positive control because it is a well-known antagonist of TRPA1 receptor. The antagonists were incubated for 10 minutes at room temperature. The plate was transferred to a FlexStation 3 microplate reader (Molecular devices LLC, San Jose, USA) with a second plate containing the activator of TRPA1 receptor: AITC (allyl isothiocyanate from Sigma Aldrich) at 10µM. The FlexStation 3^{®} transferred automatically AITC into each well and read the calcium flow in real time.

### Results:

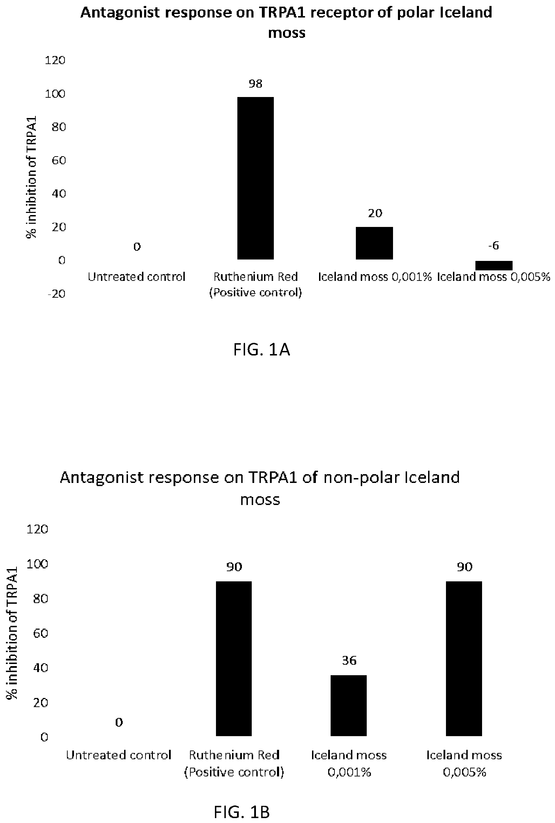

The results are shown in FIGS. 1A-B. As seen in FIG. 1A the polar extract exhibited 20% inhibition at a concentration of 0.001% and -6% at a concentration of 0.005 %. In contrast, and as shown in FIG. 1B, the non-polar extract demonstrated the same percent inhibition as the positive control at a concentration of 0.005 %, and 36% inhibition at a concentration of 0.001 %. The above data shows the TRPA1 activity is much greater for the non-polar extract compared to the polar extract. These results are surprising because no literature has suggested a difference a difference between non-polar extract compared to the polar extract for Iceland moss.

### EXAMPLE 2 - Non-Polar Iceland Moss Extract Cytotoxicity on CHO (Chinese Hamster Ovary Cell Line)

The cytotoxicity of the non-polar Iceland moss extract was assessed on CHO cell in one replicate at 2 concentrations (0.005% and 0.001%) for 24 hours. The cytotoxicity test is performed after 24 hours of incubation by the distribution of 0.2 mL of MTT solution (methylthiazolyldiphenyl-tetrazolium bromide, Sigma Aldrich, reference M2128) at 0.5 mg/mL in medium per well. Then, the cells were incubated for 3 hours at 37°C and isopropanol (200 µl) was added. The plate was shaken for 30 min and read at 570nm. Values are considered to indicate cytotoxicity if below 80% cell viability compared to an untreated control.

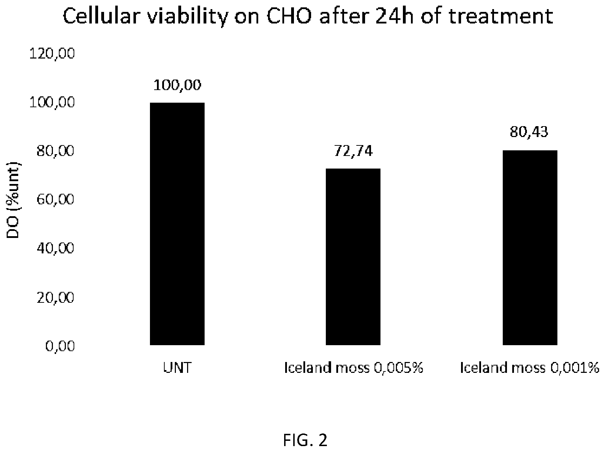

The results of the test are shown in FIG. 2. As can be seen, the non-polar Iceland moss extract was not cytotoxic at 0.001% .

### EXAMPLE 3 - Non-Polar Iceland Moss Extract TrkA Antagonistic Activity

The antagonistic activity of non-polar Iceland moss extract on the TrkA receptor was tested using the PathHunter^{®} eXpress Receptor Tyrosine Kinase (RTK) Functional Assay kit (Discoverx), which provides a cell-based functional assay for monitoring receptor tyrosine kinase activation.

At day 0, cells were plated at 30.000 cells/ wells in 80µL of cell plating reagent n°16 (See PathHunter^{®} eXpress kit, Discoverx) and incubated (37°C) overnight. The cells were then treated with 20 µL of non-polar Iceland moss extract concentrated 5 times to obtain the desired final concentration in the well (final volume in the well = 100 µL) or with a positive control. The positive control was a known antagonist GW441756 of TrKA receptor (Enzolife sciences, reference: BML-EI364-0010) (Montagnoli C, Pistilli A, Stabile A M, et al. Anti-proliferative effects of GW441756, a novel inhibitor of NGF receptor tyrosine kinase a (TRKA), in human sarcoma. Italian Journal of Anatomy and Embryology, 2010, 115(1/2): 117). The samples were incubated for 1 hour at 37 °C.

20 µl of NGF (Nerve growth factor) at a concentration of 4 nM, a well-known agonist of TrKA receptor was added and incubated 180 min at room temperature. Then, 50 µL of Pathhunter^{®} reagent were added according to the manufacturer's instructions: mix of 1 mL of substrate reagent 1, 5 mL of substrate reagent 2 and 19 mL of cell assay buffer. and incubated for 1 hour at room temperature. The chemiluminescent signal was then read on an EnVision^{®} microplate reader (Perkin Elmer, Waltham, USA) at 540 nm.

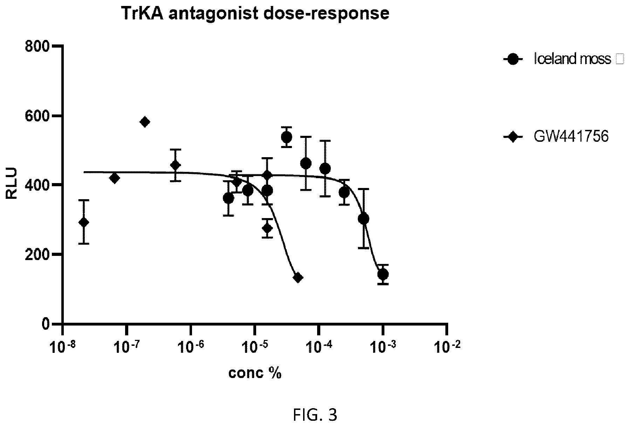

The results are shown in FIG. 3. As can be seen from the figure, the non-polar Iceland moss extract showed a dose dependent TrKA antagonist activity. The IC₅₀ (half maximal inhibitory concentration) was determined to be 0.0005284 % for the non-polar Iceland moss extract and 2.230 x 10⁻⁵ % for GW441756. These results are surprising because it is not possible to predict which compounds exhibit this effect.

### EXAMPLE 4 - Validation of Non-Polar Iceland Moss Extract TrKA Antagonistic Activity by PC 12 Neurite Outgrowth Functional Model

The antagonistic activity of non-polar Iceland moss extract on the TrkA receptor was tested using a PC12 Neurite Outgrowth Functional model according to the following process:
Day 0: 12-well plate coating with collagen.
   A collagen solution from rat tail (Sigma Aldrich, reference: 122-20) to enhance cell attachment and proliferation was reconstituted at 20µg/ml. 1 ml per well was distributed and incubated for 3 hours at room temperature. After removing the solution, the plates were dried at room temperature overnight.
Day 1: PC12 cell seeding and treatment with TrKA antagonists
   1ml of PC12 cells (a cell line derived from a pheochromocytoma of the rat adrenal medulla, at 50.000 cells/ ml were seeded in DMEM F12 (Dulbecco's Modified Eagle Medium: Nutrient Mixture F-12) with 10% FCS (Fetal calf serum)) were added per well. The cells were incubated for 24 hours at 37 °C. Then the culture medium was changed without rinsing and replaced by a basal medium containing DMEM-F12, 25ng/ml NGF to induce neurite outgrowth, insulin 5µg/ml, hydrocortisone (10ng/ml) and B27 50X, a supplement for neuronal cell culture, diluted at 1/10 (Thermo scientific, reference: 17504044). This basal medium was used as the control condition. Then, different treatments were added to this medium:
   - GW441756 at a concentration of 0.00006% (equivalent at 2µM)
   - Non-polar Iceland moss extract at a concentration of 0.0005%
   - Non-polar Iceland moss extract at a concentration of 0.0001%

The treated cells were left in culture for 5 days and then, pictures were taken (5 random pictures per well). The neurite outgrowths of PC12 cells was quantified by image analysis through the software ImageJ^{®} (module NeuronJ). Briefly, the length of each neurite was measured, and mean neurite length was then calculated for each condition.
The experiment was repeated three times and a statistical analysis with ANOVA was performed.

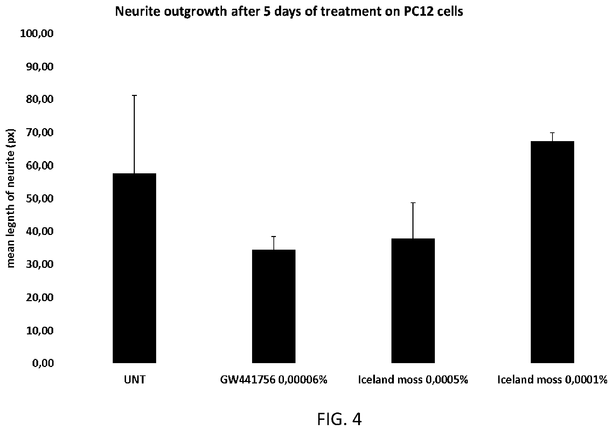

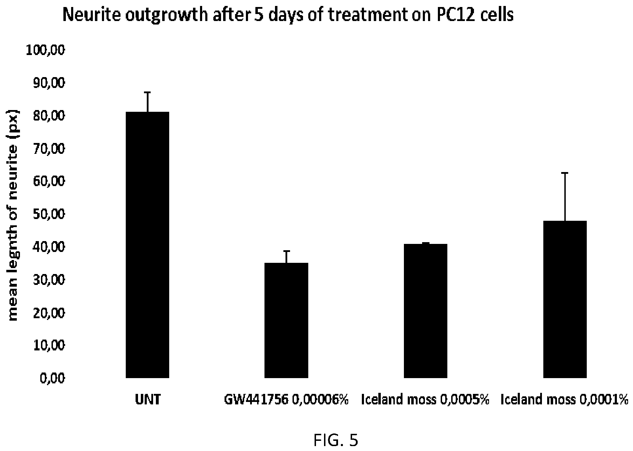

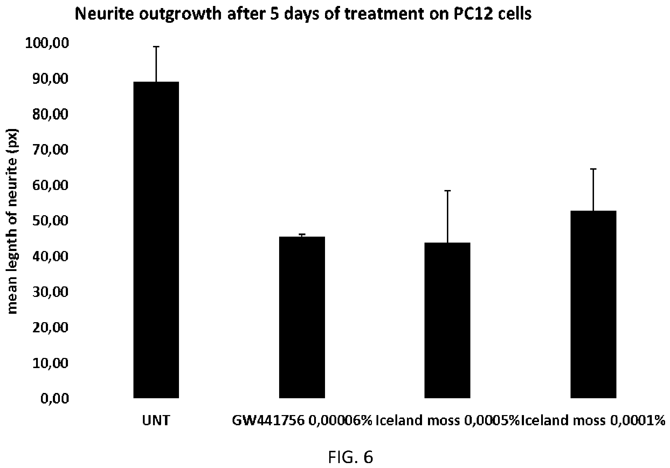

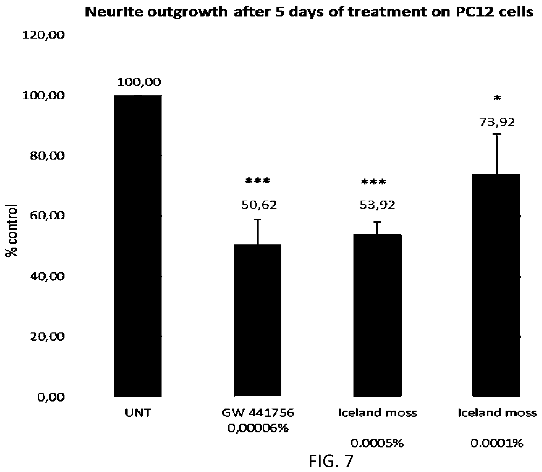

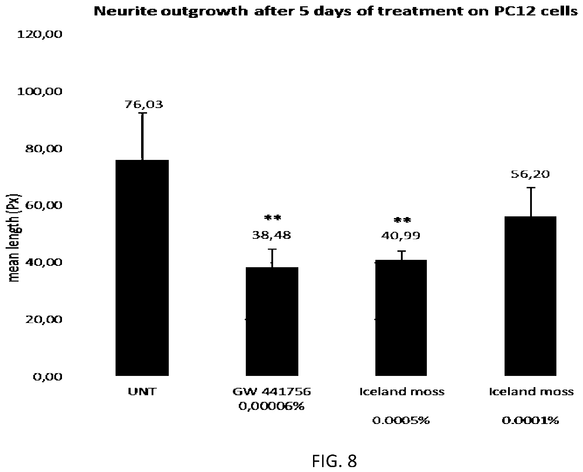

The results of each of the three experiments is shown in FIGS. 4-6. The values represented in FIGS 4-6 show the neuronal length which is lower in the case where TrKA receptor is inhibited. Expressed in pixel, this is extracted from an image (picture) taken from the culture plate. The smaller the neurite length is, the higher the inhibition of growth. The mean of the three experiments is shown in FIG. 7-8. In FIG. 7, the results are expressed in percentage of the untreated control and in FIG. 8, the results are expressed in neuronal length (pixel). As seen from the results, a significant decrease of neurite outgrowth was observed when the PC12 cells were treated with non-polar Iceland moss extract, particularly at a concentration 0.0005 %, as compared to the positive control. These results are surprising because Iceland moss shows a similar TrKA antagonist activity compared to the positive control at a very low concentration (10-fold concentration between positive compound which a pure molecule and the non-polar Iceland moss extract).

## Claims

1. A composition comprising a therapeutically effective dose of non-polar extract of Iceland moss for use in reducing pain or itch to a patient in need thereof.

2. The composition of claim 1, wherein the non-polar extract was extracted with one or more solvents selected from the group consisting of Ci-Cs alkanes, C₁-C₈ cycloalkanes, C₁-C₈ alkyl ethers, petroleum ethers, C₁-C₈ ketones, methylene chloride, ethyl acetate, xylene, toluene, chloroform, vegetable oil, mineral oil, and combinations thereof, preferably the non-polar extract was extracted with dichloromethane.

3. The composition of claim 1 or 2, wherein the pain is TRPA1 or TrKA receptor-mediated pain and/or itch.

4. The composition according to any of claims 1 to 3, wherein the composition is administered enterally, applied to a surface of skin of the patient, or is administered to the eye of the patient.

5. The composition according to any of claims 1 to 3, wherein the pain or itch is associated with digestive health, ocular itch, osteoarthritis, allergy, or atopic dermatitis.

6. The composition according to any of the preceding claims, wherein the composition further comprises a dermatologically acceptable carrier selected from the group consisting of surfactants, chelating agents, emollients, humectants, conditioners, preservatives, opacifiers, fragrances, and combinations of two or more thereof.

7. The composition according to any of the preceding claims, wherein the composition is administered in the form of a solution, suspension, lotion, cream, serum, gel, stick, spray, ointment, liquid wash, soap bar, shampoo, hair conditioner, paste, foam, powder, mousse, shaving cream, hydrogel, or film-forming product.

8. The composition according to any of the preceding claims, wherein the non-polar extract is present in the composition at a concentration of about 0.00001 to about 2 wt.% by weight of the total composition, preferably at a concentration of about 0.001 to about 0.005 wt.% by weight of the total composition.

9. A composition comprising a therapeutically effective dose of non-polar extract of Iceland moss for use in reducing pain or itch to a patient in need thereof
wherein the non-polar extract is present in the composition at a concentration of about 0.001 to about 0.005 wt.% by weight of the total composition; and
the non-polar extract was extracted with dichloromethane.

10. The composition of claim 9, wherein the pain is TRPA1 or TrKA receptor-mediated pain and/or itch.

11. The composition of claim 9 or 10, wherein the composition is applied to a surface of skin of the patient, or is administered to the eye of the patient.

12. The composition of claim 9 or 10, wherein the composition is administered enterally to the patient.

13. The composition of claim 9 or 10, wherein the pain or itch is associated with digestive health, ocular itch, osteoarthritis, allergy, or atopic dermatitis.

14. The composition according to any of claims 9 to 13, wherein the composition further comprises a dermatologically acceptable carrier selected from the group consisting of surfactants, chelating agents, emollients, humectants, conditioners, preservatives, opacifiers, fragrances, and combinations of two or more thereof.

15. The composition according to any of claims 9 to 14 , wherein the composition is administered in the form of a solution, suspension, lotion, cream, serum, gel, stick, spray, ointment, liquid wash, soap bar, shampoo, hair conditioner, paste, foam, powder, mousse, shaving cream, hydrogel, or film-forming product.
